(19) Europäisches Patentamt
European Patent Office
Office européen des brevets

(11) **EP 4 292 598 A1**

(12) **EUROPEAN PATENT APPLICATION**

(43) Date of publication:
**20.12.2023 Bulletin 2023/51**

(21) Application number: **23163851.1**

(22) Date of filing: **23.03.2023**

(51) International Patent Classification (IPC):
*A61K 35/19* (2015.01)  *A61K 8/98* (2006.01)
*A61K 9/19* (2006.01)  *A61K 38/18* (2006.01)
*F26B 5/06* (2006.01)  *A61P 43/00* (2006.01)

(52) Cooperative Patent Classification (CPC):
**A61P 43/00; A61K 8/983; A61K 9/19; A61K 35/19;
A61K 38/1808; A61K 38/1825; A61K 38/1841;
A61K 38/1858; A61K 38/1866; F26B 5/06;**
A61K 2800/84

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **16.06.2022 US 202217841941
29.12.2022 TW 111150685**

(71) Applicants:
• **Spirit Scientific Co. LTD.
22175, Taiwan (TW)**
• **Lin, Dao Lung, Steven
New Taipei City 22175 (TW)**

(72) Inventors:
• **CHEN, Chin-Ho
22175 Taiwan (TW)**
• **LIN, Dao Lung Steven
22175 Taiwan (TW)**

(74) Representative: **Haseltine Lake Kempner LLP
Cheapside House
138 Cheapside
London EC2V 6BJ (GB)**

Remarks:
A request for correction of Description has been filed pursuant to Rule 139 EPC. A decision on the request will be taken during the proceedings before the Examining Division (Guidelines for Examination in the EPO, A-V, 3.).

(54) **A GROWTH FACTOR ENRICHED DRY POWDER AND THE USE THEREOF FOR RELIEVING INFLAMMATION OR INJURY**

(57)    The present invention provides a use of a growth factors-enriched dry powder for relieving inflammation or injury, wherein each gram of the growth factors-enriched dry powder comprises more than or equal to $9 \times 10^4$ pg of PDGF-BB.

**EP 4 292 598 A1**

**Description**

**[FIELD OF THE INVENTION]**

The present invention relates to a growth factors-enriched dry powder, and in particular to the use of a growth factors-enriched dry powder for relieving inflammation or injury.

**[BACKGROUND OF THE INVENTION]**

Inflammation is the body's defense mechanism. Inflammatory reactions are often triggered in various parts of the body due to infections, traumas or hypersensitivity in order to remove harmful irritants and pathogens to promote tissue repairing. Inflammation can be divided into acute inflammation and chronic inflammation such as dermatitis, allergic rhinitis, pneumonia, hepatitis and enteritis. Dermatitis can be divided into Irritant contact dermatitis (ICD) and allergic contact dermatitis; Severe pneumonia can lead to disease such as Chronic Obstructive Pulmonary Disease (COPD) and pulmonary fibrosis; while severe hepatitis can lead to disease such as liver cirrhosis and cancer.

Platelet-Rich Plasma (PRP) is a blood-derived product with concentrations approximately 1.5 to 5 times higher than normal blood. Platelets contain platelet-related growth factors, such as Platelet-Derived Growth Factor (PDGF), Vascular Endothelial Growth Factor (VEGF), Epidermal Growth Factor (EGF), Fibroblast Growth Factor (FGF), and Transforming Growth Factor Beta 1 (TGF-$\beta$1).

Although Platelet-Rich Plasma (PRP) has anti-inflammatory properties (Reference 1, 2), clinical studies have found that Platelet-Rich Plasma (PRP) is ineffective in suppressing inflammation. The reason for this is that the low concentration and unstable quality of growth factors in Platelet-Rich Plasma (PRP), and it is not easy to preserve; moreover, in allogeneic clinical applications, Platelet-Rich Plasma (PRP) can easily trigger allergic reactions, thus increasing the risk of clinical use.

There is an urgent need in the market for a pharmaceutical composition capable of breaking through the limitation of current technological to relieve or treat inflammation or injury in various parts.

**[Summary of The Invention]**

In view of the defects from the prior art, the objective of the present invention is intended to provide a growth factors-enriched dry powder that can effectively relieve or treat the degree of inflammation or injury.

Another objective of the present invention is to provide a method for preparing a growth factors-enriched dry powder as described.

A furter objective of the present invention is to provide a growth factors-enriched dry powder for use in relieving or treating the degree of inflammation or injury described.

It is also an objective of the present invention to provide the preparation of a pharmaceutical composition or health composition for use in relieving or treating the degree of inflammation or injury.

A method of manufacturing a growth factors-enriched dry powder comprising: (a) taking a unit of a platelet-containing solution and performing a process of purifying platelets to obtain ultra-pure platelet solution; (b) performing an activation procedure to allow platelets activation in ultra-pure platelet solution and release growth factors to obtain a platelet growth factors-extract solution; (c) performing a procedure to remove a platelet-associated structure to obtain a growth factors-enriched solution; and (d) subjecting the growth factors-enriched solution to a drying process to obtain the growth factors-enriched dry powder.

In some embodiments, the drying process is a freeze-drying process. In some embodiments, the drying process is a freeze-drying process, and the freeze-drying process comprises several steps which freeze the multiple components of the growth factors-enriched solution and cause at least one of the frozen components to sublimate directly into a gaseous state and escape, thereby (1) forming multiple pores (e.g., multiple gas-entrapping pores or multiple gas-evacuation pores) within the growth factors-enriched dry powder, or (2) producing a change sufficient to cause an alteration in the physiological response through the process of converting a growth factors-enriched solution to a growth factors-enriched dry powder. In some embodiments, the frozen components comprise a frozen solute, a frozen platelet-related growth factors, and a plurality of other frozen components; and the frozen component that is directly sublimated into a gaseous state are (1') frozen solute or (1") frozen solute and at least one other frozen component. In some embodiments, the morphology of the multiple pores (e.g., multiple gas-entrapping pores or multiple gas-evacuation pores) within the growth factors-enriched dry powder is observed by electron microscopy or other means.

In some embodiments, the drying process is a freeze-drying process, a low temperature drying process, or a vacuum drying process.

In some embodiments, the process of purifying platelets in step (a) further comprises a procedure for removing white blood cells so that the number of white blood cells per milliliter (mL) of the ultra-pure platelet solution is less than

$10^6$ cells.

In some embodiments, the process of purifying platelets in step (a) further comprises a procedure for removing white blood cells so that the number of white blood cells per milliliter (mL) of the ultra-pure platelet solution is less than 50 , 100 , 200 , 300 , 400 , 500 , 600 , 700 , 800 , 900 , 1,000 , 2,000 , 3,000 , 4,000 , 5,000 , 6,000 , 7,000 , 8,000 , 9,000 , 10,000 , 20,000 , 30,000 , 40,000 , 50,000 , 60,000 , 70,000 , 80,000 , 90,000 , 100,000 , 200,000 , 300,000 , 400,000 , 500,000 , 600,000 , 700,000 , or 800,000 cells.

In some embodiments, the process of purifying platelets in step (a) further comprises a procedure for removing plasma so that the concentration of albumin in the ultra-pure platelet solution is less than 3 g/dL; in some embodiments, the process of purifying platelets further comprises a procedure for removing plasma so that the concentration of globulin in the ultra-pure platelet solution is less than 2 g/dL; in some embodiments, the process of purifying platelets further comprises a procedure for removing plasma so that the concentration of fibrinogen in the ultra-pure platelet solution is less than 100 $\mu$g/mL.

In some embodiments, the process of purifying platelets in step (a) further comprises a procedure for removing plasma so that the concentration of albumin in the ultra-pure platelet solution is less than 0.25 , 0.5 , 0.75 , 1 , 1.25 , 1.5 , 1.75 , 2 , 2.25 , 2.5 , or 2.75 g/dL.

In some embodiments, the process of purifying platelets further comprises a procedure for removing plasma so that the concentration of globulin in the ultra-pure platelet solution is less than 0.25 , 0.5 , 0.75 , 1 , or 1.25g/dL.

In some embodiments, the process of purifying platelets further comprises a procedure for removing plasma so that the concentration of fibrinogen in the ultra-pure platelet solution is less than 10 , 20 , 30 , 40 , 50 , 60 , 70 , 80 or 90 $\mu$g/mL.

In some embodiments, the platelet-containing solution is whole blood, and step (a) comprises: (a1) taking a unit of platelet-containing solution; (a2) performing the first sedimentation process, so that the platelet-containing solution is separated into a plasma layer, a buffy coat layer, and a red blood cells layer; (a3) Removing the buffy coat layer and the red blood cells layer to obtain a Plasma layer solution; (a4) performing a second sedimentation process, which allows platelets to settle and form a pellet; (a5) removing the supernatant and mixing the pellet with a solvent to obtain the ultra-pure platelet solution.

In some embodiments, the activation procedure is a freezing and then thawing procedure (i.e., a freeze-thaw procedure), an addition of $Ca^{2+}$ procedure, an addition of thrombin procedure, an addition of collagen procedure, or an addition of ADP procedure, or any combination thereof.

In some embodiments, the activation procedure is a freezing and then thawing procedure (i.e., freeze-thaw procedure), and the freezing and then thawing procedure (i.e., freeze-thaw procedure) comprises (b1) placing a container containing the ultra-pure platelet solution in liquid nitrogen to freeze the ultra-pure platelet solution; and (b2) raising the temperature of the container to thaw the frozen ultra-pure platelet solution.

In some embodiments, the content of PDGF-BB per gram (g) of growth factors-enriched dry powder is 10 pg ~ $3 \times 10^7$pg.

In some embodiments, the content of PDGF-BB per gram (g) of growth factors-enriched dry powder is 10 , 20 , 30 , 40 , 50 , 60 , 70 , 80 , 90 , $1 \times 10^2$ , $5 \times 10^2$ , $1 \times 10^3$ , $5 \times 10^3$ , $1 \times 10^4$ , $5 \times 10^4$ , $1 \times 10^5$ , $5 \times 10^5$ , $1 \times 10^6$ , $5 \times 10^6$ , or $1 \times 10^7$pg.

In some embodiments, the content of VEGF per gram (g) of growth factors-enriched dry powder is $2 \times 10^2$pg ~ $3 \times 10^6$ pg.

In some embodiments, the content of VEGF per gram (g) of growth factors-enriched dry powder is $4 \times 10^2$ , $8 \times 10^2$ , $1 \times 10^3$ , $5 \times 10^3$ , $1 \times 10^4$ , $5 \times 10^4$ , $1 \times 10^5$ , $5 \times 10^5$ , or $1 \times 10^6$ pg.

In some embodiments, the content of FGF per gram (g) of growth factors-enriched dry powder is 10 pg ~ $1 \times 10^4$pg.

In some embodiments, the content of FGF per gram (g) of growth factors-enriched dry powder is 10 , 20 , 30 , 40 , 50 , 60 , 70 , 80 , 90 , $1 \times 10^2$ , $5 \times 10^2$ , $1 \times 10^3$ , or $5 \times 10^3$pg.

In some embodiments, the content of TGF-$\beta$1 per gram (g) of growth factors-enriched dry powder is $2 \times 10^5$pg ~ $1.2 \times 10^7$pg.

In some embodiments, the content of TGF-$\beta$1 per gram (g) of growth factors-enriched dry powder is $3 \times 10^5$ , $5 \times 10^5$ , $1 \times 10^6$ , $5 \times 10^6$ , or $1 \times 10^7$pg.

In some embodiments, the content of EGF per gram (g) of growth factors-enriched dry powder is $1 \times 10^2$pg ~ $6 \times 10^5$pg.

In some embodiments, the content of EGF per gram (g) of growth factors-enriched dry powder is $2 \times 10^2$ , $5 \times 10^2$ , $1 \times 10^3$ , $5 \times 10^3$ , $1 \times 10^4$ , $5 \times 10^4$ , $1 \times 10^5$ , or $5 \times 10^5$pg.

In some embodiments, the freeze-drying procedure comprises lowering the temperature to less than or equal to -35°C and lowering the pressure to less than or equal to 80 mTorr.

In some embodiments, the freeze-drying procedure comprises lowering the temperature to less than or equal to -40, -45, -50, -60, -65, or -70°C.

In some embodiments, the freeze-drying procedure comprises lowering the pressure to less than or equal to 70, 60, 50, 40, or 30 mTorr.

In some embodiments, the platelet-containing solution is whole blood, apheresis platelet, leukocytes-reduced platelets apheresis, or Platelet-Rich Plasma (PRP), or any combination thereof; or the platelet-containing solution is derived from an autologous blood sample or an allogeneic blood sample, or a combination thereof.

In some embodiments, the solvent in step (a5) is sterile saline, water for injection, 0.45% NaCl, 4.5% hypertonic saline, sterile warm spring water, or isotonic saline; or, the platelet concentration in the ultra-pure platelet solution in step (a5) is $1\times10^9$ cells/mL $\sim 10\times10^9$ cells/mL.

In some embodiments, the platelet concentration in the ultra-pure platelet solution in step (a5) is $2\times10^9$ cells/mL, $3\times10^9$ cells/mL, $4\times10^9$ cells/mL, $5\times10^9$ cells/mL, $6\times10^9$ cells/mL, $7\times10^9$ cells/mL, $8\times10^9$ cells/mL, or $9\times10^9$ cells/mL.

In some embodiments, step (c) comprises: (c1) performing a sedimentation process to allow a platelet-associated structure to settle and form a platelet-associated structure pellet; (c2) removing the platelet-associated structure pellet to obtain the growth factors-enriched solution.

The present invention further provides a growth factors-enriched dry powder that is prepared by a preparation method, and the preparation method comprises: (a) taking a unit of platelet-containing solution and performing a process of purifying platelets to obtain an ultra-pure platelet solution; (b) performing an activation procedure to allow platelets activation in ultra-pure platelet solution and release growth factors to obtain a platelet growth factors-extract solution; (c) performing a platelet-associated structure removal procedure to obtain a growth factors-enriched solution; and (d) performing the freeze-drying procedure on growth factors-enriched solution in order to obtain the growth factors-enriched dry powder, wherein the freeze-drying procedure comprises lowering the temperature to less than or equal to -30°C and lowering the pressure to less than or equal to 100 mTorr; wherein, the content of PDGF-BB per gram (g) of growth factors-enriched dry powder is 10 pg $\sim 3\times10^7$ pg.

In some embodiments, the activation procedure is a freezing and then thawing procedure (i.e., a freeze-thaw procedure), and the freezing and then thawing procedure (i.e., a freeze-thaw procedure) comprises: (b1) placing a container containing the ultra-pure platelet solution in liquid nitrogen to freeze the ultra-pure platelet solution; and (b2) raising the temperature of the container to thaw the frozen ultra-pure platelet solution.

The invention further provides for the use of a growth factors-enriched dry powder in the preparation of a pharmaceutical composition or health composition for administrating an effective dose to a specific site in an individual to relieve the degree of inflammation or injury in the site.

In some embodiments, the individual is a human or other mammal.

In some embodiments, the activation procedure is a freezing and then thawing procedure (i.e., a freeze-thaw procedure), and the freezing and then thawing procedure (i.e., a freeze-thaw procedure) comprises: (b1) place a container containing the ultra-pure platelet solution in liquid nitrogen to freeze the ultra-pure platelet solution; and (b2) raise the temperature of the container to thaw the frozen ultra-pure platelet solution.

In some embodiments, the process of purifying platelets in step (a) further comprises a procedure for removing white blood cells so that the number of white blood cells per milliliter (mL) of the ultra-pure platelet solution is less than $10^6$ cells.

In some embodiments, the process of purifying platelets in step (a) further comprises a procedure for removing plasma so that the concentration of albumin in the ultra-pure platelet solution is less than 3 g/dL; or, the process of purifying platelets comprises a procedure to remove plasma so that the concentration of globulin in the ultra-pure platelet solution is less than 2 g/dL; or, the process of purifying platelets comprises a procedure to remove plasma so that the concentration of fibrinogen in the ultra-pure platelet solution is less than 100 μg/mL.

In some embodiments, the content of VEGF per gram (g) of growth factors-enriched dry powder is $2\times10^2$ pg $\sim 3\times10^6$ pg.

In some embodiments, the content of FGF per gram (g) of growth factors-enriched dry powder is 10pg $\sim 1\times10^4$ pg.

In some embodiments, the content of TGF-β1 per gram (g) of growth factors-enriched dry powder is $2\times10^5$ pg $\sim 1.2\times10^7$ pg.

In some embodiments, the content of EGF per gram (g) of growth factors-enriched dry powder is $1\times10^2$ pg $\sim 6\times10^5$ pg.

In some embodiments, the growth factors-enriched dry powder in the preparation of a pharmaceutical composition or health composition to relieve the degree of inflammation or injury of a respiratory tract, skin, muscles, tendons, bones, joints, or ligaments of the individual.

**[BRIEF DESCRIPTION OF THE DRAWING]**

Fig. 1: Flow chart of preparing growth factors-enriched dry powder.
Fig. 2A : The histogram of the effects of different activation methods on the release multiples of growth factors.
Fig. 2B : The histogram of the effects of different freezing-thaw procedures on the release multiples of growth factors.
Fig. 3A : The histogram of the effect of growth factors-enriched dry powder on human dermal fibroblast proliferation.
Fig. 3B : The histogram of the effect of growth factors-enriched dry powder on relieving inflammation of human dermal fibroblasts.
Fig. 4 : The histogram of the effect of different types of growth factors-enriched dry powder solutions on relieving inflammation in human dermal fibroblasts.
Fig. 5 : The histogram of the effect of freeze-drying procedure on the anti-inflammatory effect of the product.
Fig. 6 : The histogram of the effect of platelet concentration on the total amount of growth factors in the preparation procedure.

**[DETAILED DESCRIPTION OF THE INVENTION]**

The following is a detailed description of the embodiments of the present invention, as well as the technology and features of the present invention. However, this embodiment is not intended to limit the present invention, and any changes and modifications made by anyone who is familiar with the technology without departing from the spirit and scope of the present invention should be included in the scope of the patent application of the present invention.

In the present invention, the growth factors-enriched dry powder refers to the dry powder rich in platelet-related growth factors such as Platelet-Derived Growth Factor (PDGF) enriched dry powder, Vascular Endothelial Growth Factor (VEGF) enriched dry powder, Epidermal Growth Factor (EGF) enriched dry powder, Fibroblast Growth Factor (FGF) enriched dry powder, or Transforming Growth Factor Beta 1 (TGF-$\beta$1) enriched dry powder, or any combination thereof.

In the present invention, "enriched platelet-related growth factors" dry powder has a higher concentration of platelet-related growth factors than the source composition from which the dry powder is isolated.

In the present invention, the growth factors-enriched solution refers to the solution rich in platelet-related growth factors, such as Platelet-Derived Growth Factor (PDGF) enriched solution, Vascular Endothelial Growth Factor (VEGF) enriched solution, Epidermal Growth Factor (EGF) enriched solution, Fibroblast Growth Factor (FGF) enriched solution, or Transforming Growth Factor Beta 1 (TGF-$\beta$1) enriched solution, or any combination thereof.

In the present invention, an "enriched platelet-related growth factors" solution has a higher concentration of platelet-related growth factors than the source solution from which the solution is isolated.

In the present invention, "one unit" of a platelet-containing solution refers to the capacity of a container of any size for a platelet-containing solution, such as the capacity of a blood bag for a platelet-containing solution is 250 mL, the capacity of a 50 mL centrifuge tube for a platelet-containing solution is 50 mL, the capacity of a 1 mL container for a platelet-containing solution is 1 mL.

In the present invention, ultra-pure platelet solution refers to a higher purity platelet solution purified from a platelet-containing solution.

In the present invention, the platelet-associated structure refers to platelets, platelet fragments, platelet structures formed by aggregation between platelets, or any combination thereof.

In the present invention, the sedimentation process refers to the process of depositing and precipitating substances, such as the process of depositing and precipitating substances by using gravity, centrifugal force, or electromagnetic force.

In the present invention, "health composition" or "health product" refers to a nutritional supplement; a composition that can promote subject's tissues to maintain normal physiological functions after being administered to an subject, or can relievesubject's symptoms after being administered to ansubject.

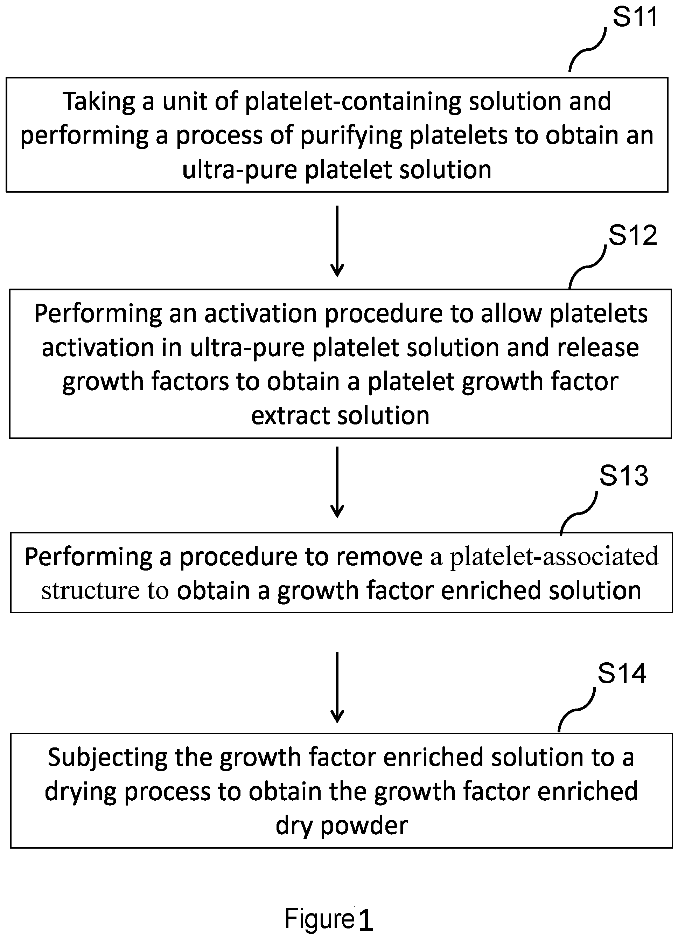

Please refere to Figure 1, the preparation of growth factors-enriched dry powder comprises the following steps:

(S11)taking a unit of platelet-containing solution and performing a process of purifying platelets to obtain an ultra-pure platelet solution ;
(S12)performing an activation procedure to allow the platelets activation in ultra-pure platelet solution and release growth factors to obtain a platelet growth factor extract solution ;
(S13)performing a procedure to remove a platelet-associated structure to obtain a growth factors-enriched solution ; and
(S14)subjecting the growth factors-enriched solution to a drying process to obtain the growth factors-enriched dry powder .

In some embodiments, the platelet-containing solution is whole blood, apheresis platelet, leukocytes-reduced platelets apheresis, or Platelet-Rich Plasma (PRP), or any combination thereof. In some embodiments, the platelet-containing solution is derived from an autologous blood sample or an allogeneic blood sample, or a combination thereof.

In some embodiments, step (S11) further comprises sub-steps:

(S111) taking a unit of platelet-containing solution; and
(S112) performing at least one sedimentation process to separate the platelet-containing solution into multiple layers; wherein, the multiple layers comprise a platelet layer (e.g., the first platelet layer), and a white blood cell layer. The white blood cell layer is removed to obtain the ultra-pure platelet solution, and makes the number of white blood cells in the ultra-pure platelet solution per milliliter (mL) less than $10^6$. Or, performing at least one sedimentation process to separate the platelet-containing solution into multiple layers, wherein, the multiple layers include a platelet layer (e.g., the second platelet layer), and plasma and plasma protein layer. The plasma and plasma protein layer are removed to obtain the ultra-pure platelet solution, and make the concentration of albumin in the ultra-pure platelet solution less than 3g/dL, or make the concentration of globulin in ultra-pure platelet solution less than 2g/dL, or make the concentration of fibrinogen in ultra-pure platelet solution less than 100$\mu$g/mL.

In some embodiments, step (S112) further comprises sub-steps:

(S1121) performing the first sedimentation process, so that the platelet-containing solution is separated into a first platelet layer (e.g., a plasma layer), a white blood cell layer (e.g., a buffy coat layer), and red blood cells layer;
(S1122) removing the buffy coat layer and the red blood cells layer to obtain a plasma layer solution; (S1123) performing the second sedimentation process to allow the platelets to settle and form a pellet, which is the second platelet layer, and the supernatant is the plasma and plasma protein layer; and
(S1124) removing the supernatant, and mixing the pellet with a solvent to obtain the ultra-pure platelet solution.

In some embodiments, the solvent is sterile saline, water for injection, 0.45% NaCl, 4.5% hypertonic saline, sterile warm spring water, or isotonic saline, or any combination thereof. In some embodiments, the platelet concentration in the ultra-pure platelet solution is $1\times10^9$/mL ~ $10\times10^9$/mL.

In some embodiments, the lysis procedure in step (S12) is a freezing and then thawing procedure (i.e., freezing-thaw procedure), an addition of $Ca^{2+}$ procedure, an addition of thrombin procedure, an addition of collagen procedure, or an addition of ADP procedure, or any combination thereof.

In some embodiments, the activation procedure in step (S12) is a freezing and then thawing procedure (i.e., a freeze-thaw procedure), and step (S12) further comprises sub-steps:

(S121) placing a container containing the ultra-pure platelet solution in liquid nitrogen to freeze the ultra-pure platelet solution; and
(S122) raising the temperature of the container to thaw the frozen ultra-pure platelet solution, thereby allow the platelet activation in ultra-pure platelet solution and release growth factors to obtain the platelet growth factor extract solution.

In some embodiments, step (S13) further comprises sub-steps:

(S131) performing a sedimentation process to allow the platelet-associated structure to settle and form a platelet-associated structure pellet; and
(S132) removing platelet-associated structure pellets to obtain the growth factors-enriched solution.

In some embodiments, in step (S14), the freeze-drying procedure comprises lowering the temperature to less than or equal to -30°C and lowering the pressure to less than or equal to 100 mTorr. In some embodiments, in step (S14), the freeze-drying procedure comprises lowering the temperature to less than or equal to -35°C and lowering the pressure to less than or equal to 80 mTorr.

Embodiment 1: Preparation of growth factors-enriched dry powder, and analyze the growth factor concentration thereof.

Embodiment 1-1: Preparation of growth factors-enriched dry powder.

In this embodiment, the following steps (S11') ~(S14') are used to prepare growth factors-enriched dry powder:

(S11') performing the procedure for preparing the ultra-pure platelet solution, which comprised the following sub-steps:

(S111') placing 250 mL of human whole blood into a blood bag containing anticoagulant;

(S112') performing the process of purifying platelets, which comprises the following sub-steps:

(S1121') the human whole blood is centrifuged (500~1,200g, 5~8 minutes; this is the first centrifugation) using a centrifuge to separate human whole blood into three layers; from top to bottom were plasma layer, buffy coat layer, where white blood cells are located, and red blood cells layer (see references 3-6). The stratification of blood cells after centrifugation is well known to those skilled in the art, and the centrifugation conditions for the first centrifugation can be expanded to 300-1,500 g for 3~10 minutes. After centrifugation, platelets are distributed in the plasma layer and adjacent to the buffy coat layer.

(S1122') Aseptically aspirate the plasma layer to completely remove white blood cells and red blood cells, platelet-rich plasma (PRP) in present invention is then obtained, which is also known as plasma layer solution in this invention;

(S1123') Detecting the total number of platelets in the plasma layer solution (Plasma layer solution) using an Automatic Hemocytometer Analyzer (model XP-300, SYSMEX brand). The plasma layer solution is centrifuged (1000 ~ 2,500g, 5 minutes; this is the second centrifugation) using a centrifuge, to allow platelets to settle and form a pellet that is the platelet layer, and the supernatant is plasma and plasma protein layer. The above-mentioned centrifugation conditions for the second centrifugation can be expanded to 500-3,000g, 5-20 minutes, and the centrifugation speed must be higher than the actual centrifugation speed (300-1,500g, 3-10 minutes) used in the first centrifugation step above;

(S1124') Completely removing the supernatant to totally take away the plasma and the protein in plasma, and then suspending the platelets in the platelet layer with water for injection (to make the platelet concentration $1\times10^9$/mL; 10-20 mL in total) to obtain ultra-pure platelet solution.

(S12') Performing an activation procedure, which comprises the following sub-steps:

(S121') placing the cryotube containing the ultra-pure platelet solution into liquid nitrogen and allow it stand for 20-30 minutes (the freezing time can be extended to 5-120 minutes), to freeze the ultra-pure platelet solution; wherein, the cryotube contains $1\times10^9$ platelets, and the platelet concentration in the ultra-pure platelet solution is $1\times10^9$/mL.

(S122') Placing the cryotube in a 37°C water bath to thaw the frozen ultra-pure platelet solution, thereby allow the platelets activation in the ultra-pure platelet solution and released growth factors to obtain the platelet growth factor extract solution.

(S13') performing a procedure for removing platelet-associated structures, which compriseed the following sub-steps:

(S131') the platelet growth factor extract solution is centrifuged using a centrifuge (10,000~15,000g, 5~10 minutes) to allow the platelet-associated structure to settle and form a platelet-associated structure pellet. Centrifugation conditions can be expanded to 8,000-20,000g, 3-15 minutes.

(S132') Carefully transfer the supernatant into the specimen bottle to completely remove the platelet-associated structure pellet, the supernatant is the growth factors-enriched solution. Wherein, the specimen bottle contains about 1 mL of growth factors-enriched solution, and this about 1 mL of growth factors-enriched solution contains growth factors released by $1\times10^9$ platelets.

(S14') performing a freeze-drying (freeze-drying) procedure, comprising the following sub-steps:

(S141') pre-cooling (-30~-50°C, at least 5 hours);

(S142') first drying (the temperature rises from the temperature gradient of step S141' to 10°C for 10-20 hours, and the vacuum value is less than or equal to 100 mTorr); and

(S143') Secondary drying (20°C, 3~5 hours, vacuum value less than or equal to 100 mTorr). The dried product after freeze-drying is the growth factors-enriched dry powder.

From the above preparation procedure, the preparation procedure of the growth factors-enriched dry powder comprises the step of removing white blood cells (WBC) and plasma, and the step of stimulating platelets (e.g., by freezing and thawing), the step of removing platelet-associated structure, and step of drying (e.g., via freezing, cryogenic, or vacuum drying steps). Therefore, the growth factors-enriched dry powder comprises low concentration or even no white blood cells (WBC), plasma, and platelets.

In some embodiments, the growth factors-enriched dry powder comprises low concentration or even no albumin.

In some embodiments, the growth factors-enriched dry powder comprises low concentration or even no globulin.

In some embodiments, the growth factors-enriched dry powder comprises low concentration or even no fibrinogen.

Embodiment 1-2: Analysis of composition of growth factors-enriched dry powder.

Using an analytical balance (ME204 model, Mettler brand) to measure the weight of 3 bottles of growth factors-enriched dry powder. Mixing 3 bottles of growth factors-enriched dry powder with 1 mL of normal saline respectively to obtain 3 bottles of growth factors-enriched dry powder solution/suspension. An Automated Hematology Analyzer (XP-300 model, SYSMEX brand) is used to analyze the number of white blood cells (WBC) in each bottle of growth factors-enriched dry powder solution/suspension, and use a microscope and counting methods to detect the number of platelets in growth factors-enriched dry powder solution/suspension; then use enzyme-linked immunosorbent assay (ELISA) to analyze the concentration of various growth factors (including PDGF-BB, VEGF, EGF, FGF, TGF-$\beta$1) in growth factors-enriched dry powder solution/suspension. Subsequently, use the various growth factor concentrations, white blood cell (WBC) concentration, and platelet concentration in the growth factors-enriched dry powder solution/suspension to deduce the weight of various growth factors, the number of white blood cells (WBC), and the number of platelets per gram (g) of growth factors-enriched dry powder solution/suspension. The experimental results are shown in Table 1.

Table 1

| Growth factors-enriched dry powder test items | mean $\pm$ standard deviation |
| --- | --- |
| Growth factors-enriched dry powder Net weight (g) | 0.0030$\pm$0.0001 |
| The number of platelets (number/ng) | 0-1,000 |
| The amount of white blood cells (WBC) (number/ng) | 0-100 |
| PDGF-BB content (pg) of one gram (g) growth factors-enriched dry powder | 3,446,200$\pm$556,140 |
| VEGF content (pg) of one gram (g) growth factors-enriched dry powder | 379,787.01$\pm$14,884.36 |
| EGF content (pg) of one gram (g) growth factors-enriched dry powder | 253,430$\pm$22,210 |
| FGF content (pg) of one gram (g) growth factors-enriched dry powder | 4,415.05$\pm$463 |
| TGF-$\beta$1 content (pg) of one gram (g) growth factors-enriched dry powder | 819,530$\pm$127,480 |

As shown in Table 1, the weight of each bottle of growth factors-enriched dry powder is 0.0030 grams (g) on average. The growth factors-enriched dry powder comprises 3,446,200pg of PDGF-BB, 379,787.01pg of VEGF, 253,430pg of EGF, 4,515.05pg of FGF, and 819,530pg of TGF-$\beta$1 per gram (g) on average; moreover, either no white blood cells (or about 0-100 white blood cells (WBC)) and no platelets (or about 0-1,000 platelets) can be detected in growth factors-enriched dry powder per nanogram (ng) on average.

Embodiment 2: Effect of freezing and thawing (activation procedure) on the release factor of growth factors.

Perform the step of Experiment 1-1 (S 11') to prepare an ultra-pure platelet solution and the platelet concentration in the ultra-pure platelet solution is $1\times10^9$/mL. Then, the ultra-pure platelet solution is divided into two groups, i.e., the refrigerated group and the Frozen-thawed group.
Frozen-thawed group: take 1mL of the ultra-pure platelet solution, and perform the steps (S12') ~ (S14') of Embodiment 1-1 to prepare the dry powder of the frozen-thawed group (that is, the growth factors-enriched dry powder in this invention).
Refrigerated group: take 1mL of the ultra-pure platelet solution, and not perform the activation procedure in step (S12') of Embodiment 1-1; i.e., the ultra-pure platelet solution is not placed in liquid nitrogen to freeze and then thaw, but only refrigerated at 4°C. Then, perform the steps (S13') ~ (S14') of Embodiment 1-1 to prepare the dry powder of the refrigerated group.
The dry powder of the refrigerated group and the dry powder of the frozen-thawed group were respectively mixed with 1mL of water for injection to prepare the refrigerated group dry powder solution and the frozen-thawed group dry powder solution. Then, they used the enzyme-linked immunosorbent assay (ELISA) to analyze the concentration of growth factors in each group of solutions, and they used the following formula to calculate the multiples of growth factors released from each group.
The multiple of growth factor released in the frozen-thawed group = (the growth factor concentrations of the solution in the frozen-thawed group $\times$ the volume of the solution in the frozen-thawed group) $\div$ (the growth factor concentrations of the solution in the refrigerated group $\times$ the volume of the solution in the refrigerated group)
The multiple of growth factor released in the refrigerated group = (the growth factor concentrations of the solution in the refrigerated group $\times$ the volume of the solution in the refrigerated group) $\div$ (the growth factor concentrations of the solution in refrigerated group $\times$ the volume of the solution in the refrigerated group solution)

Table 2

| Group | The multiple of growth factor released (fold) |
|---|---|
| Refrigerated group | 1 |
| Frozen-thawed group | 1.8~15 |

Please refer to the experimental results shown in Table 2. As shown in Table 2, the total amount of growth factors in the dry powder solution of the frozen-thawed group is 1.8-15 folds that of the dry powder solution in the refrigerated group. What can be seen is that the activation procedure of freezing and then thawing has an unexpected effect of significantly increase the release of growth factors by 1.8 to 15 folds.

Embodiment 3: The effect of different activation methods on the multiples of growth factors released.

Embodiment 3-1: The effect of different activation methods on the multiples of growth factors released.

Perform the step (S11') of Embodiment 1-1 to prepare an ultra-pure platelet solution, and the platelet concentration in the ultra-pure platelet solution is $1\times10^9$/mL. Then, the ultra-pure platelet solution is divided into control group and 4 experimental groups, namely calcium ion group, collagen group, adenosine diphosphate group, and -196°C (liquid nitrogen) group.

Control group: take 1mL of the ultra-pure platelet solution, and instead of performing the activation procedure in the step (S12') of the embodiment 1-1, the steps (S13') ~ (S14') of the embodiment 1-1 is performed directly to prepare dry powder of growth factors without the activation procedure; and then mix with 1mL of water for injection, which is the control group solution.

-196°C (liquid nitrogen) group: take 1mL of the ultra-pure platelet solution, and perform the steps (S12') ~ (S14') of Embodiment 1-1 to prepare the -196°C (liquid nitrogen) group dry powder (that is, the growth factors-enriched dry powder in present invention); then, mix with 1 mL of water for injection to prepare -196°C (liquid nitrogen) group solution.

Calcium ion group: take 1mL of the ultra-pure platelet solution, and not perform the activation procedure in the step (S12') of the Embodiment 1-1; that is, the ultra-pure platelet solution is not placed in liquid nitrogen to freeze and then thaw, but mix with calcium chloride to make the calcium ion concentration in the mixture reach 4.54 mg/mL, and let it stand for 20-30 minutes (the activation time can be expanded to 5-120 minutes, and the standing time is the same as that of the -196°C (liquid nitrogen) group when performing the step (S121')). Subsequently, perform the steps (S13') and (S14') of Embodiment 1-1 to prepare the calcium ion group dry powder, and then mix it with 1 mL of water for injection to prepare the calcium ion group solution.

Collagen group: take 1mL of the ultra-pure platelet solution, and not perform the activation procedure in the step (S12') of the Embodiment 1-1; that is, the ultra-pure platelet solution is not placed in liquid nitrogen to freeze and then thaw; instead, mix with collagen (C7661, Sigma-Aldrich brand) to make the collagen concentration in the mixture reach 25ug/mL, and let it stand for 20-30 minutes (the activation time can be expanded to 5-120 minutes, and the standing time is the same as that of the -196°C (liquid nitrogen) group when performing the step (S121')). Subsequently, perform the steps (S13') and (S14') of Embodiment 1-1 to prepare the collagen group dry powder, and then mix it with 1 mL of water for injection to prepare the collagen group solution.

Adenosine diphosphate (ADP) group: take 1mL of the ultra-pure platelet solution, and not perform the activation procedure in the step (S12') of the Embodiment 1-1; that is, the ultra-pure platelet solution is not placed in liquid nitrogen to freeze and then thaw; instead, mix with adenosine diphosphate (ADP) to make the adenosine diphosphate (ADP) concentration in the mixture reaches 0.8mM, and let it stand for 20-30 minutes, (the activation time can be expanded to 5-120 minutes, and the standing time is the same as that of the -196°C (liquid nitrogen) group when performing the step (S121')). Subsequently, perform the steps (S13') and (S14') of Embodiment 1-1 to prepare the adenosine diphosphate (ADP) group dry powder, and then mix it with 1 mL of water for injection to prepare the adenosine diphosphate (ADP) group solution.

The enzyme-linked immunosorbent assay (ELISA) is used to analyze the concentration of growth factors in each group of solutions, and the following formula is used to calculate the multiples of growth factors released from each group.

The multiple of growth factor released in the control group = (the growth factor concentrations of the solution in the control group $\times$ the volume of the solution in the control group) $\div$ (the growth factor concentrations of the solution in the control group $\times$ the volume of the solution in the control group).

The multiple of growth factor released in the experimental group = (the growth factor concentrations of the solution in the experimental group × the volume of the solution in the experimental group) ÷ (the growth factor concentrations of the solution in the control group × the volume of the solution in the control group).

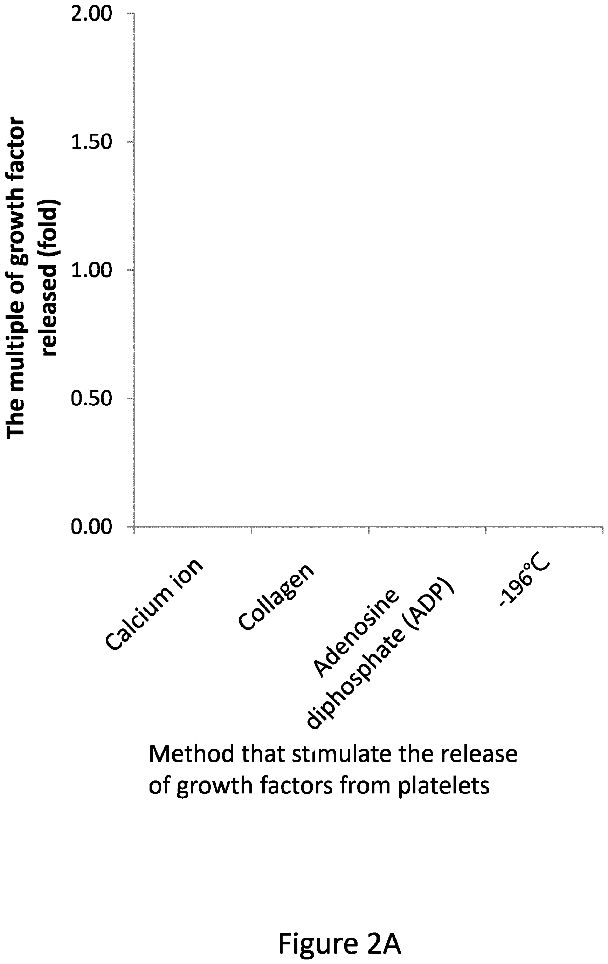

The experimental results are shown in Table 3 and Figure 2A.

Table 3

| Groups | The multiple of growth factor released (fold) |
|---|---|
| Calcium ion group | 0.63 |
| Collagen group | 0.23 |
| Adenosine diphosphate (ADP) group | 0.19 |
| -196°C (liquid nitrogen) group | 1.81 |

Please refer to Figure 2A. Figure 2A is the histogram of the effects of different activation methods on the release multiples of growth factors.

As the results are shown in Table 3 and Figure 2A, the total amount of growth factors in calcium ion group solution, collagen group solution, and adenosine diphosphate group solution is 0.63 fold, 0.23 fold, and 0.19 fold of the total amount of growth factor in control group solution respectively; this demonstrates that these treatment methods can make the total amount of growth factors drop significantly. Unexpectedly, the total amount of growth factors in the -196°C (liquid nitrogen) group solution is 1.8 folds that of the control group solution; i.e., the activation procedure of freezing at -196°C (liquid nitrogen) and then thawing has an unexpected effect of significantly increase the release of growth factors to 2.9 ~ 9.5 times (1.8 ÷ 0.63 ≒2.9; 1.8 ÷ 0.19 ≒9.5).

Embodiment3-2: Effects of different freezing-thaw procedures on the release multiples of growth factors.

Perform the step (S11') of Embodiment 1-1 to prepare an ultra-pure platelet solution, and the platelet concentration in the ultra-pure platelet solution is $1 \times 10^9$/mL. Then, the ultra-pure platelet solution is divided into a control group and 5 experimental groups, namely -20°C group, -30°C group, -40°C group, -80°C group, and -196°C (liquid nitrogen) group.

Control group: take 1mL of the ultra-pure platelet solution, and instead of performing the activation procedure in the step (S12') of the embodiment 1-1, the steps (S13') ~ (S14') of the embodiment 1-1 is performed directly to prepare dry powder of growth factors without the activation procedure; and then mix with 1mL of water for injection, which is the control group solution.

-196°C (liquid nitrogen) group: take 1mL of the ultra-pure platelet solution, and perform the steps (S12') ~ (S14') of Embodiment 1-1 to prepare the -196°C (liquid nitrogen) group dry powder (that is, the growth factors-enriched dry powder in this invention).

-20°C group: take 1mL of the ultra-pure platelet solution, and not perform the activation procedure in the step (S12') of the Embodiment 1-1; that is, the ultra-pure platelet solution is not placed in liquid nitrogen to freeze and then thaw. Instead, it is placed in a -20°C refugirater to freeze and than thaw. Then, perform the steps (S13') and (S14') of Embodiment 1-1 to prepare the -20°C dry powder.

-30°C group: take 1mL of the ultra-pure platelet solution, and not perform the activation procedure in the step (S12') of the Embodiment 1-1; that is, the ultra-pure platelet solution is not placed in liquid nitrogen to freeze and then thaw. Instead, it is placed in a -30°C refugirater to freeze and than thaw. Then, perform the steps (S13') and (S14') of Embodiment 1-1 to prepare the -30°C dry powder.

-40°C group: take 1mL of the ultra-pure platelet solution, and not perform the activation procedure in the step (S12') of the Embodiment 1-1; that is, the ultra-pure platelet solution is not placed in liquid nitrogen to freeze and then thaw. Instead, it is placed in a -40°C refrigerator to freeze and than thaw. Then, perform the steps (S13') and (S14') of Embodiment 1-1 to prepare the -40°C dry powder.

-80°C group: take 1mL of the ultra-pure platelet solution, and not perform the activation procedure in the step (S12') of the Embodiment 1-1; that is, the ultra-pure platelet solution is not placed in liquid nitrogen to freeze and then thaw. Instead, it is placed in a -80°C refrigerator to freeze and then thaw. Then, perform the steps (S13') and (S14') of Embodiment 1-1 to prepare the -80°C dry powder.

Mix -20°C dry powder, -30°C dry powder, -40°C dry powder, -80°C dry powder, and -196°C (liquid nitrogen) dry powder with 1mL of water for injection respectively to prepare -20°C dry powder solution, -30°C dry powder solution, -40°C dry powder solution, -80°C dry powder solution, and -196°C (liquid nitrogen) dry powder solution. Then the enzyme-linked immunosorbent assay (ELISA) is used to analyze the concentration of growth factors in each group of solutions, and the following formula is used to calculate the multiples of growth factors released from each group.

The multiple of growth factor released in the control group = (the growth factor concentrations of the solution in the control group × the volume of the solution in the control group) ÷ (the growth factor concentrations of the solution in the control group × the volume of the solution in the control group).

The multiple of growth factor released in the experimental group = (the growth factor concentrations of the solution in the experimental group × the volume of the solution in the experimental group) ÷ (the growth factor concentrations of the solution in the control group × the volume of the solution in the control group).

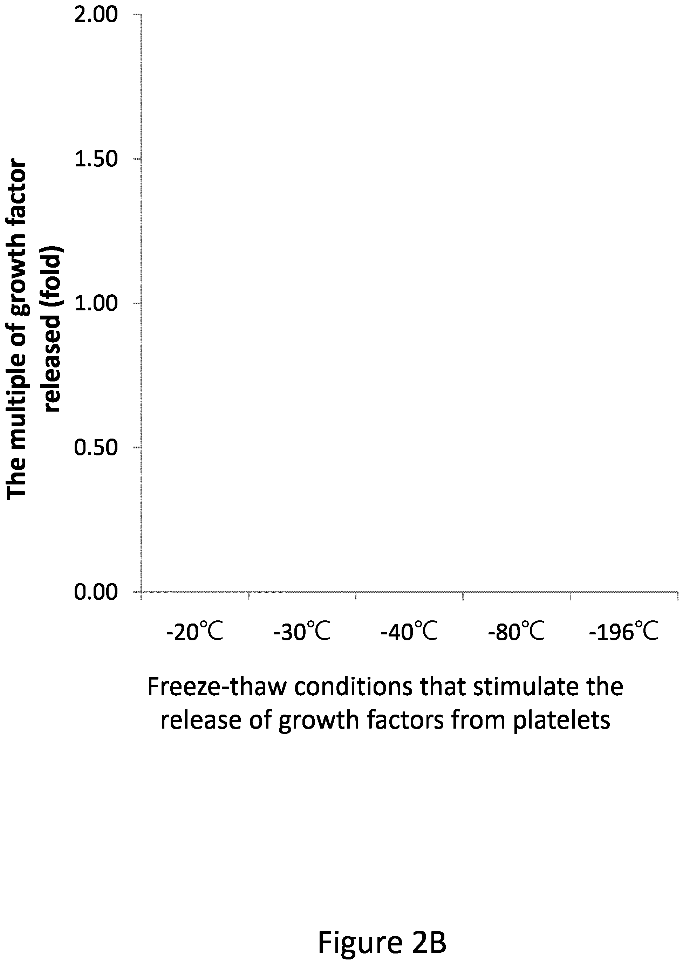

The experimental results are shown in Table 4 and Figure 2B.

Table 4

| Group | The multiple of growth factor released (fold) |
|---|---|
| -20°C group | 1.04 |
| -30°C group | 0.97 |
| -40°C group | 0.88 |
| -80°C group | 1.02 |
| -196°C group | 1.81 |

Please refer to Figure 2B. Figure 2B is the histogram of the effects of different freezing-thaw procedures on the release multiples of growth factors.

As the results are shown in Table 4 and Figure 2B, the total amount of growth factors in -20°C group solution, -30°C group solution, -40°C group solution and -80°C group solution is 1.04 folds, 0.97 fold, 0.88 fold and 1.02 folds of the total amount of growth factor in control group solution respectively; this demonstrates that the total amount of growth factors obtained by freezing and then thawing at -20°C to -80°C is $1 \pm 0.12$ folds that of the non-freezing-thaw group (control group). i.e., freezing- thaw procedure performed at -20°C to -80°C is unable to significantly increase the total amount of growth factors. Unexpectedly, the total amount of growth factors in the -196°C (liquid nitrogen) group solution is 1.8 folds that of the control group solution; i.e., the activation procedure of freezing at -196°C (liquid nitrogen) and then thawing has an unexpected effect of significantly increase the release of growth factors from 1 to 1.8 folds.

Embodiment 4: Effects of growth factors-enriched dry powder on tissue repair and anti-inflammation.

Embodiment 4-1: Effects of growth factors-enriched dry powder on tissue repair.

Human dermal fibroblast (HDF) (purchased from ScienCell Research Laboratories, Inc., product number 2320) is cultured in Dulbecco's Modified Eagle medium (DMEM) (Corning 10-013CV) containing 1% human platelet lysate (HPL), and then replaced with fresh medium for later use. The above-mentioned human dermal fibroblast (HDF) is adherent cell.

The 96-well plate is divided into untreated wells (control wells) and growth factors-enriched dry powder wells (RD2201 wells) that are prepared according to Experiment 1-1, with 2 replicates each. Human dermal fibroblasts (HDF) are added to untreated wells (control group wells) and RD2201 wells so that each well contains the same number of cells, and place in an incubator at 37°C and 5% carbon dioxide ($CO_2$) for 24 hours.

Aspirate the used medium. Mix 1 bottle of growth factors-enriched dry powder described in Embodiment 1-2 (each bottle contains 0.0030 grams (g) of growth factors-enriched dry powder on average) with 1 mL of DMEM culture medium (containing 1% HPL) to obtain a high-concentration mixed solution; and then mix the high-concentrated mixed solution with DMEM culture medium (containing 1 % HPL) (the volume ratio of the high-concentration mixed solution to DMEM culture medium is 1: 19) to prepare growth factors-enriched dry powder medium. Suspend the cells in RD2201 wells with the growth factors-enriched dry powder medium, and suspend the cells in untreated wells (control wells) with DMEM medium (containing 1% HPL). Incubate in an incubator with 5% carbon dioxide ($CO_2$) at 37°C for 24 hours.

Aspirate the used medium. Add 90 μL DMEM and 10 μL CCK-8 buffer (DOJINDO, product number CK04) to each well. Incubate in an incubator with 5% carbon dioxide ($CO_2$) at 37°C for 3 hours. The analysis is performed using a multimode microplate reader (model Varioskan LUX, Thermo Scientific brand) with an absorbance value set at 450 nm. The regression curve of absorbance value and cell number is established according to CCK-8 buffer product manual, and then the absorbance value is converted into the number of surviving cells.

Data are presented as mean ± SD, and statistical analysis is performed by T TEST. Groups marked with "*" indicates statistical differences ($p < 0.05$) from untreated wells (control group wells).

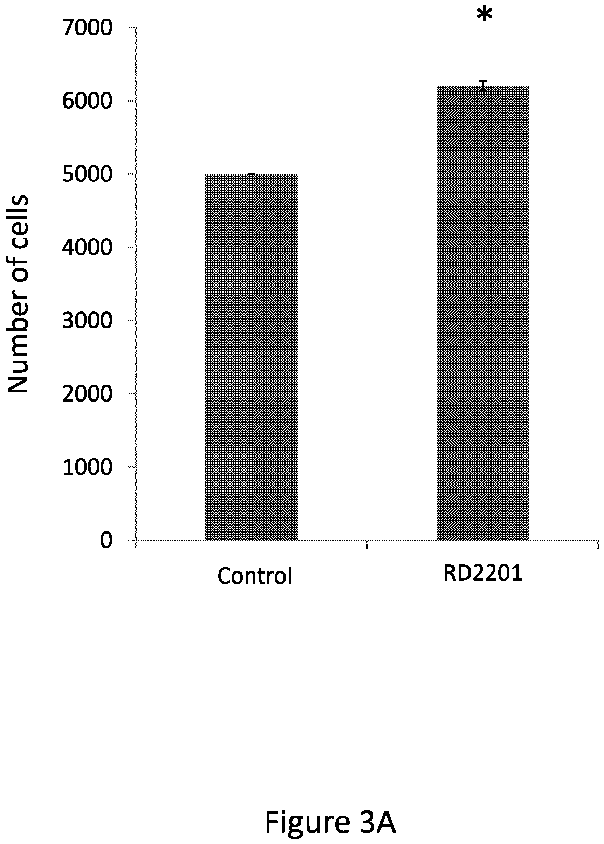

Please refer to Figure 3A. Figure 3A is the histogram of the effect of growth factors-enriched dry powder on human dermal fibroblast proliferation. What can be seen from Figure 3A is that the number of cells in the control group wells is 5,000, and the number of cells in the RD2201 group well is 6,203; and there is a significant difference between the two groups ($p<0.05$). Thus, it demonstrates that the growth factors-enriched dry powder can promote the proliferation of dermal fibroblasts, thereby promoting the dermal tissue repair. The inventor expects that the growth factors-enriched dry powder can also promote the proliferation of fibroblasts in other human tissues, thereby promoting the mentioned other tissues repair.

Embodiment 4-2: Effect of growth factors-enriched dry powder on relieving inflammatory response

Utilize the same human dermal fibroblast (HDF) as Embodiment 4-1 to conduct the experiment. Human dermal fibroblast (HDF) is cultured in Dulbecco's Modified Eagle medium (DMEM) (Corning 10-013CV) containing 2% fetal bovine serum (FBS), and then replaced with fresh medium for later use.

A 96-well plate is divided into untreated wells (control group wells) and growth factors-enriched dry powder wells (RD2201 wells) with 3 replicates each; wherein the specimens of 3 replicates are derived from whole blood of three different human individuals. Human dermal fibroblasts (HDF) are added to untreated wells (control wells) and RD2201 wells, so that each well contains the same number of cells; then incubate in an incubator with 5% carbon dioxide ($CO_2$) at 37°C for 24 hours.

Aspirate the used medium. Mix one bottle of growth factors-enriched dry powder described in Embodiment 1-2 (each bottle contains 0.0030 grams (g) of growth factors-enriched dry powder on average) with 1 mL DMEM culture medium (containing 2% FBS) to obtain a high-concentration mixed solution; and then mix the high-concentrated mixed solution with DMEM culture medium (containing 2% HPL) (the volume ratio of the high-concentration mixed solution to DMEM culture medium is 1: 19) to prepare growth factors-enriched dry powder medium. Suspend the cells in RD2201 wells with the growth factors-enriched dry powder medium, and suspend the cells in untreated wells (control wells) with DMEM culture medium (containing 2% FBS). Incubate in an incubator with 5% carbon dioxide ($CO_2$) at 37°C for 24 hours.

Transfer the cell culture medium from untreated wells (control group wells) and RD2201-treated wells into separate microcentrifuge tubes. Use interleukin-6 (IL-6, an inflammatory factor) ELISA kit (Human IL-6 ELISA Kit) (product number ab178013, Abeam brand) to detect the concentration of human interleukin-6 (Human IL-6) in the cell culture medium. Then the analysis is performed using a multimode microplate reader (model Varioskan LUX, Thermo Scientific brand) with an absorbance value set at 450 nm. The regression curve of the absorbance value and the concentration of the inflammatory factor IL-6 is established according to the product manual of the interleukin-6 (IL-6) ELISA kit, and then the absorbance value is converted into the concentration of the inflammatory factor IL-6; and use the following formula to calculate the release percentage of inflammatory factors in each group.

The release percentage of inflammatory factors in the control

group = (the concentration of inflammatory factor IL-6 in the control group ÷

the concentration of inflammatory factor IL-6 in the control group) × 100%.

The release percentage of inflammatory factors in the RD2201

group = (the concentration of the inflammatory factor IL-6 in the RD2201 group

÷ the concentration of the inflammatory factor IL-6 in the control group) ×

100%.

Data are presented as mean ± SD, and statistical analysis is performed by T TEST. Groups marked with "*" indicates statistical differences ($p < 0.05$) from untreated wells (control group wells).

Table 5

| Group | Release percentage (%) of inflammatory factors | | | |
|---|---|---|---|---|
| | First replicate | Second replicate | Third replicate | Average |
| Control group | 100 | 100 | 100 | 100 |
| RD2201 group | 13.33 | 16.67 | 80.95 | 36.98 |

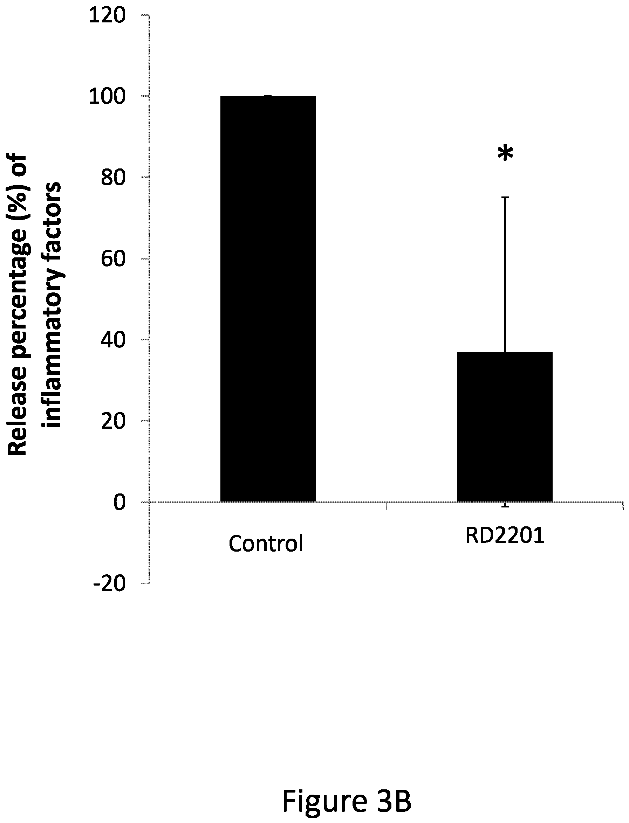

Please refer to Table 5 and Figure 3B. Figure 3B is the histogram of the effect of growth factors-enriched dry powder on relieving inflammation of human dermal fibroblasts in this invention. The results of Table 5 and Figure 3B show that the average release percentage of inflammatory factors in the control group is 100%; the average release percentage of inflammatory factors in the RD2201 group is 36.98%, which is significantly different from that in the control group ($p < 0.05$), and the decline reaches 63%. What can be seen is that the growth factors-enriched dry powder can inhibit the inflammatory response of human dermal fibroblasts, thereby relieving the inflammation of the skin. The inventor expects that after using stimulants (such as LPS) to induce the inflammatory response of fibroblasts in other human tissues, the growth factors-enriched dry powder can also inhibit the induced inflammatory response, thereby relieving the inflammation of other tissues.

Embodiment 5: Effects of different kinds of growth factors-enriched dry powders on relieving inflammation

Human dermal fibroblasts (HDF) require for this experiment are prepared according to the experimental procedure of Embodiment 4-2. A 96-well plate is divided into untreated wells (control group wells), growth factors-enriched dry powder wells (RD2201 wells), and plasma and RD2201 mixture wells with 3 replicates each; wherein the specimens of 3 replicates are derived from whole blood of three different human individuals. Adding human dermal fibroblasts (HDF) to untreated wells (control wells), RD2201 wells, and plasma and RD2201 mixture wells, so that each well contains the same number of cells; and incubate in an incubator with 5% carbon dioxide ($CO_2$) at 37°C for 24 hours.
Aspirate the used medium. Take a blood sample and perform the steps of embodiment 1-1 to obtain 2 bottles of growth factors-enriched dry powder with the same weight. Mix one bottle of growth factors-enriched dry powder with 1mL of plasma (that is, the solution of plasma and plasma protein layer obtained in step S1123') to prepare a growth factors-enriched plasma solution. Mix another bottle of growth factors-enriched dry powder with 1 mL of DMEM culture medium (containing 2% FBS) to prepare growth factors-enriched medium. Then each group then perform the following steps individually.
Untreated wells (control group wells): The cells in untreated wells (control wells) are suspended in DMEM culture medium (containing 2% FBS), and cultured in an incubator with 5% carbon dioxide ($CO_2$) at 37°C for 24 hours.

RD2201 wells: the growth factors-enriched culture medium is mixed with DMEM culture medium (containing 2% FBS) (the volume ratio of the growth factors-enriched culture medium to DMEM culture medium is 1: 19) to obtain growth factors-enriched dry powder culture medium (the growth factors-enriched dry powder concentration is $1.5 \times 10^{-4}$ g/mL), and the cells in the RD2201 wells are suspended with the growth factors-enriched dry powder culture medium, and the cells are cultured in an incubator with 5% carbon dioxide ($CO_2$) at 37°C for 24 hours.

Plasma and RD2201 mixture wells: the growth factors-enriched plasma solution is mixed with DMEM culture medium (containing 2% FBS) (the volume ratio of the growth factors-enriched plasma solution to DMEM culture medium is 1: 19) to obtain growth factors-enriched dry powder and plasma culture medium (the dry powder concentration of growth factor is $1.5 \times 10^{-4}$ g/mL), and use to suspend the cells in the plasma and RD2201 mixture wells; then culture in an incubator with 5% carbon dioxide ($CO_2$) at 37°C for 24 hours.

Transfer the cell culture medium from untreated wells (control group wells), RD2201 wells, and the plasma and RD2201 mixture wells into separate microcentrifuge tubes. Use interleukin-6 (IL-6, an inflammatory factor) ELISA kit (Human IL-6 ELISA Kit) (product number ab178013, Abeam brand) to detect the concentration of human interleukin-6 (Human IL-6) in the cell culture medium. Then the analysis is performed using a multimode microplate reader) (model Varioskan LUX, Thermo Scientific brand) with an absorbance value set at 450 nm. The regression curve of the absorbance value and the concentration of the inflammatory factor IL-6 is established according to the product manual of the interleukin-6 (IL-6) ELISA kit, and then the absorbance value is converted into the concentration of the inflammatory factor IL-6; and use the following formula to calculate the release percentage of inflammation in each group.

The release percentage of inflammatory factors in the control group = (the concentration of inflammatory factor IL-6 in the control group ÷ the concentration of inflammatory factor IL-6 in the control group) × 100%.

Release percentage of inflammatory factors in RD2201 group = (concentration of inflammatory factor IL-6 in RD2201 group ÷ concentration of inflammatory factor IL-6 in control group) × 100%.

The release percentage of inflammatory factors in the plasma and RD2201 mixture group = (the concentration of inflammatory factor IL-6 in the plasma and RD2201 mixture group ÷ the concentration of inflammatory factor IL-6 in the control group) × 100%.

Data are presented as mean ± SD, and statistical analysis is performed by T TEST. Group marked with "+" indicates statistical difference ($p < 0.05$) from the growth factors-enriched dry powder (RD2201) well, and the group marked with "*" indicates statistical difference ($p < 0.05$) from the untreated well (control group well).

Table 6

| Group | Release percentage (%) of inflammatory factors | | | |
|---|---|---|---|---|
| | First replicate | Second replicate | Third replicate | Average |
| Control group | 100 | 100 | 100 | 100 |
| RD2201 group | 13.33 | 16.67 | 80.95 | 36.98 |
| Plasma and RD2201 mixture group | 553.33 | 206.25 | 723.81 | 494.46 |

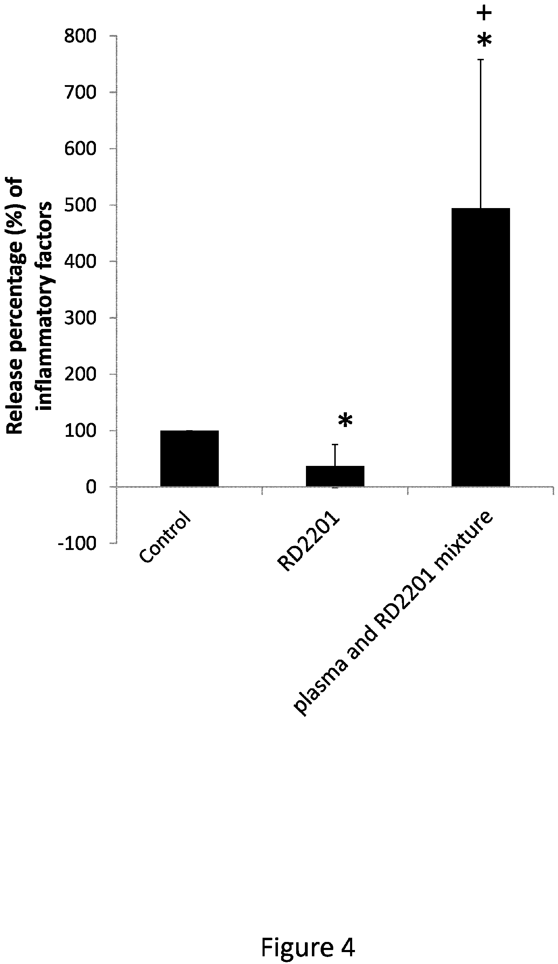

Please refer to Figure 4. Figure 4 is the histogram of the effect of different types of growth factors-enriched dry powder solutions on relieving inflammation in human dermal fibroblasts. The results in Figure 4 show that the average release percentage of inflammatory factors in the control group is 100%, the average release percentage of inflammatory factors in the RD2201 group is 36.98%, and the average release percentage of inflammatory factors in the plasma and RD2201 mixture group is 494.46%. Compare with the control group, the release percentage of inflammatory factors in the RD2201 group decrease significantly ($p<0.05$), and the decline reaches 63%; while the release percentage of inflammatory factors in the plasma and RD2201 mixture group is significantly increased ($p<0.05$), and the incline is as high as 394%. Compare with the RD2201 group, the release percentage of inflammatory factors in the plasma and RD2201 mixture group also increases significantly ($p<0.05$), and the incline is as high as 457%. From the above experiments, what can be seen that the growth factors-enriched dry powder solution containing plasma and plasma protein layer not only unable to relieve the inflammation of human dermal fibroblasts effectively, but will greatly aggravate the inflammation, Therefore, the step of removing plasma and plasma protein in present invention is an important technical feature, and the product can greatly relieve the inflammatory response, which has unexpected effects.

The inventor expects to use stimulants (such as LPS) to induce inflammatory responses in other human tissues before conducting experiments, and similar results will be obtained; i.e., the step of removing plasma and plasma protein in present invention can make the product greatly relieve the induced inflammatory reaction, and then relieve the inflammation of the tissue.

Embodiment 6: The effect of freeze-drying procedure on the anti-inflammatory effect of the product.

Human dermal fibroblasts require for this experiment are prepared according to the experimental procedure of Embodiment 4-2. The 96-well plate is divided into untreated wells (control group wells), growth factors-enriched dry powder wells (RD2201 wells), and growth factors-enriched solution wells (RD2201 without freeze-drying wells) with 3 replicates each; wherein the specimens of 3 replicates are derived from the whole blood of three different human individuals. Human dermal fibroblasts (HDF) are added to untreated wells (control group), RD2201 wells, and RD2201 without freeze-drying wells, so that each well contained the same number of cells, and incubate in an incubator with 5% carbon dioxide ($CO_2$) at 37°C for 24 hours.

Aspirate the used medium. The steps (S11') ~ (S13') of Embodiment 1-1 are performed to obtain a growth factors-enriched solution. Each group then perform the following steps individually.

Untreated wells (control group wells): Suspend the cells in untreated wells (control wells) with DMEM culture medium (containing 2% FBS), and culture them in an incubator with 5% carbon dioxide ($CO_2$) at 37°C for 24 hours.

RD2201 wells: Take 1 mL of the growth factors-enriched solution, and perform the step (S14') of Embodiment 1-1 to obtain a growth factors-enriched dry powder. Mix the growth factors-enriched dry powder with 1 mL of water for injection to prepare a growth factors-enriched dry powder solution. Mix the growth factors-enriched dry powder solution with DMEM culture medium (containing 2% FBS) to obtain a growth factors-enriched dry powder culture medium (the dry powder concentration of the growth factor is $1.5 \times 10^{-4}$ g/mL), and suspend the cells in RD2201 wells with the growth factors-enriched dry powder culture medium, then culture them in an incubator with 5% carbon dioxide ($CO_2$) at 37°C for 24 hours.

RD2201 without freeze-drying wells: Mix 1mL of growth factors-enriched solution with DMEM culture medium (containing 2% FBS) to obtain a mixture (the dry powder concentration of the growth factor is $1.5 \times 10^{-4}$ g/mL), and suspend the cells in the RD2201 without freeze-drying wells are cultured in an incubator with 5% carbon dioxide ($CO_2$) at 37°C for 24 hours.

Transfer the cell culture medium in untreated wells (control group wells), RD2201 wells, and RD2201 without freeze-drying wells into microcentrifuge tubes respectively. Use interleukin-6 (IL-6, an inflammatory factor) ELISA kit (Human IL-6 ELISA Kit) (product number ab178013, Abeam brand) to detect the concentration of human interleukin-6 (Human IL-6) in the cell culture medium. Then the analysis is performed using a multimode microplate reader (model Varioskan LUX, Thermo Scientific brand) with an absorbance value set at 450 nm. The regression curve of the absorbance value and the concentration of the inflammatory factor IL-6 is established according to the product manual of the interleukin-6 (IL-6) ELISA kit, and then the absorbance value is converted into the concentration of the inflammatory factor IL-6; and use the following formula to calculate the release percentage of inflammatory factors in each group.

The release percentage of inflammatory factors in the control group = (the concentration of inflammatory factor IL-6 in the control group ÷ the concentration of inflammatory factor IL-6 in the control group) × 100%.

The release percentage of inflammatory factors in RD2201 group = (concentration of inflammatory factor IL-6 in RD2201 group ÷ concentration of inflammatory factor IL-6 in control group) × 100%.

The release percentage of inflammatory factors in the RD2201 without freeze-drying group = (the concentration of inflammatory factor IL-6 in the RD2201 without freeze-drying group ÷ the concentration of inflammatory factor IL-6 in the control group) × 100%.

Data are presented as mean ± SD, and statistical analysis is performed by T TEST. Groups marked with "*" indicates statistical differences (p< 0.05) from untreated wells (control group wells).

Table 7

| Group | Release percentage (%) of inflammatory factors | | | |
|---|---|---|---|---|
| | First replicate | Second replicate | Third replicate | Average |
| Control group | 100 | 100 | 100 | 100 |
| RD2201 group | 13.33 | 16.67 | 80.95 | 36.98 |
| RD2201 without freeze-drying group | 66.67 | 27.08 | 133.33 | 75.69 |

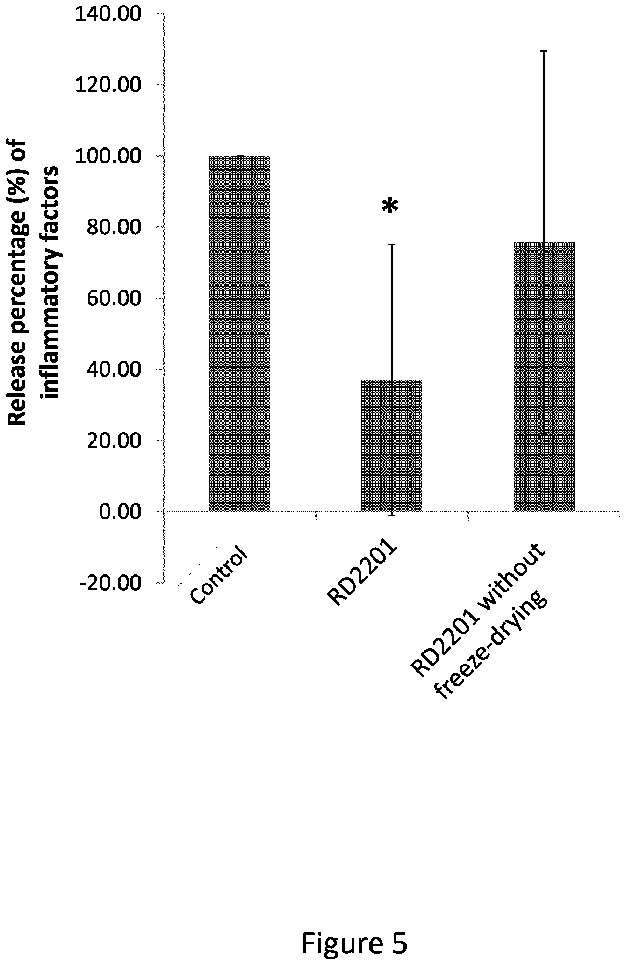

Please refer to Table 7 and Figure 5. Figure 5 is the histogram of the effect of freeze-drying procedure on the anti-inflammatory effect of the product. The results in Figure 5 show that the average release percentage of inflammatory factors in the control group is 100%, the average release percentage of inflammatory factors in the RD2201 group is 36.98%, and the average release percentage of inflammatory factors in the RD2201 without freeze-drying group is 75.69%. Compare with the control group, the release percentage of inflammatory factors in the RD2201 group is significantly decreased (p<0.05), and the decline reaches 63%. On the other hand, compare with the control group, the release percentage of inflammatory factors in the RD2201 without freeze-drying group does not decrease significantly (p>0.05). What can be seen from the above experiments is that the RD2201 without freeze-drying group is unable to relieve the inflammation of human dermal fibroblasts effectively. Therefore, the freeze-drying step in present invention is an important technical feature, and the product can greatly relieve the inflammatory response, which has unexpected effects.
The inventor expects to use stimulants (such as LPS) to induce inflammatory responses in other human tissues before conducting experiments, and similar results will be obtained; i.e., the step of freeze-drying in present invention can make the product greatly relieve the induced inflammatory response, thereby relieving the inflammation of the tissue.

Embodiment 7: Effect of platelet concentration on the total amount of growth factors during the preparation process.

In this embodiment, the platelet concentration is divided into low concentration group, medium concentration group and high concentration group. Performed the step (S11') of Embodiment 1-1 to prepare a ultra-pure platelet solution, and the platelet concentration in the ultra-pure platelet solution is $1 \times 10^9$/mL. Then, each group of dry powders is prepared in the following steps separately.

High concentration group: Take 10 mL of the ultra-pure platelet solution, and the ultra-pure platelet solution is centrifuged (1,000~2,500g, 5 minutes) using a centrifuge to allow platelets to settle and form a pellet. Remove the supernatant, and then add 1 mL of water for injection to obtain a ultra-pure platelet solution of the high concentration group (platelet concentration is $10 \times 10^9$/mL). Then, perform the steps (S12') - (S14') of Embodiment 1-1 to prepare a high concentration group dry powder.

Medium concentration group: Take 10 mL of the ultra-pure platelet solution, and the ultra-pure platelet solution is centrifuged (1,000~2,500g, 5 minutes) using a centrifuge to allow platelets to settle and form a pellet. Aspirate the supernatant, and then add 2 mL of water for injection to obtain a ultra-pure platelet solution of the medium concentration group (platelet concentration is $5 \times 10^9$/mL). Then, perform the steps (S12') - (S14') of Embodiment 1-1 to prepare a medium concentration group dry powder.

Low concentration group: Take 10 mL of the ultra-pure platelet solution, and the ultra-pure platelet solution is centrifuged (1,000~2,500g, 5 minutes) using a centrifuge to allow platelets to settle and form a pellet. Aspirate the supernatant, and then add 3 mL of water for injection to obtain a ultra-pure platelet solution of the low concentration group (platelet concentration is $3.3 \times 10^9$/mL). Then, perform the steps (S12') - (S14') of Embodiment 1-1 to prepare a low concentration group dry powder.

Mix the high concentration group dry powder, the medium concentration group dry powder, and the low concentration group dry powder with 1, 2 and 3 mL of water for injection respectively to obtain a high concentration group solution, a medium concentration group solution, and a low concentration group solution. Then, the concentration of growth factors in the three groups are analyzed by enzyme-linked immunosorbent assay (ELISA), and the total amount of growth factors released in each group is calculated by the following formula.

The total amount of growth factors released in the high concentration group = the concentration of growth factors in the high concentration group solution× the volume of the high concentration group solution.

The total amount of growth factors released in the medium concentration group = the concentration of growth factor in the medium concentration group solution × the volume of the medium concentration group solution.

The total amount of growth factors released in the low

concentration group = the concentration of growth factors in the low

concentration group solution × the volume of the low concentration group

solution.

Table 8

| Group | Number of platelets | Volume (mL) | Platelet concentration (cell/mL) | The total amount of growth factors released (ng) |
|---|---|---|---|---|
| Low concentration group | $10^{10}$ | 3 | $3.3 \times 10^9$ | 109.5 |
| Medium concentration group | $10^{10}$ | 2 | $5 \times 10^9$ | 126.3 |
| High concentration group | $10^{10}$ | 1 | $10 \times 10^9$ | 264 |

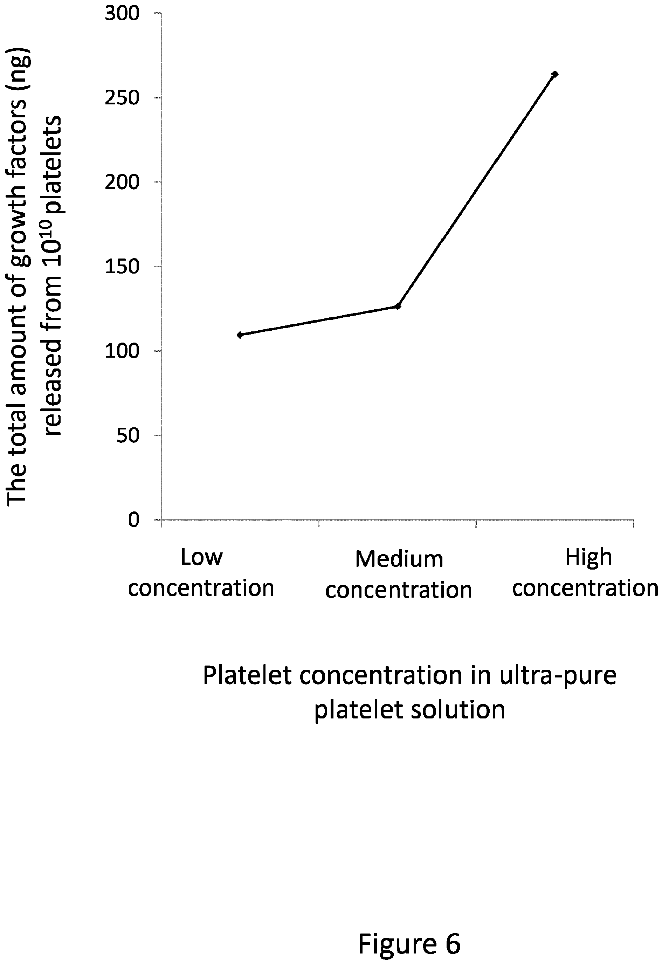

Please refer to Figure 6. Figure 6 is the histogram of the effect of platelet concentration on the total amount of growth factors in the preparation procedure. The results in Figure 6 show that when the platelet concentration in the ultra-pure platelet solution is $3.3 \times 10^9$/mL, the total amount of growth factors released is 109.5ng; when the platelet concentration in the ultra-pure platelet solution is $5 \times 10^9$/mL, the total amount of growth factors released is 126.3ng; when the platelet concentration in the ultra-pure platelet solution is $10 \times 10^9$, the total amount of growth factors released is 264ng. What can be seen from Figure 6 is that when the platelet concentration in the ultra-pure platelet solution is $5 \times 10^9$/mL~$10 \times 10^9$/mL, the total amount of growth factors released can be significantly increased, and the incline can reach 15% ~ 141%, which has unexpected effect.

The above description is only a preferred embodiment of the present invention and is not intended to limit the scope of the present invention. Therefore, all other changes or modifications shall be included within the scope of the patent application of the present invention without departing from the spirit of the invention as disclosed herein.

References

| Serial number | References |
|---|---|
| 1 | IsableAndia, Nicola Maffulli, 2013. Platelet-rich plasma for managing pain and inflammation in osteoarthritis. Nature Review Rheumatology 15, 721-730. |
| 2 | Pengcheng Xu et al., 2020. Platelet-rich plasma accelerates skin wound healing by promoting re-epithelialization. Burns & Trauma |
| 3 | Linfeng Piao, Hyungmin Park, and Chris Hyunchul Jo, 2017. Theoretical prediction and validation of cell recovery rates in preparing platelet-rich plasma through a centrifugation. PLOS ONE 12. |
| 4 | Sean R Downing, Giannoula L Klement, 2012. Isolation and proteomic analysis of platelets by SELDI-TOF MS. Methods in Molecular Biology 818: 153-170. |
| 5 | Zu-yao Chang, Gheorghe A.M. Pop and Gerard C.M. Meijer, 2004. A NOVEL MODEL OF BLOOD IMPEDANCE FOR INDIRECT VISCOSITY MEASUREMENT. |
| 6 | https://stanfordbloodcenter.org/buffy_coats/ |

**Claims**

1.  A method of manufacturing a growth factors-enriched dry powder, comprising:

    (a) taking a unit of platelet-containing solution and performing a process of purifying platelets to obtain an ultra-pure platelet solution;
    (b) performing an activation procedure to allow the platelets activation in ultra-pure platelet solution and release growth factors to obtain a platelet growth factor extract solution;
    (c) performing a procedure to remove a platelet-associated structure to obtain a growth factors-enriched solution; and
    (d) subjecting the growth factors-enriched solution to a drying process to obtain the growth factors-enriched dry powder.

2.  The method of claim 1, wherein, the drying process is a freeze-drying process.

3.  The method of claim 1, the process of purifying platelets in step (a) further comprises a process of removing white blood cells, so that the number of white blood cells per milliliter (mL) of the ultra-pure platelet solution is less than $10^6$ cells.

4.  The method of claim 1, the process of purifying platelets in step (a) further comprises a process of removing plasma, so that the concentration of albumin in the ultra-pure platelet solution is less than 3g/dL; or, the process of purifying platelets comprises a procedure to remove plasma so that the concentration of globulin in the ultra-pure platelet solution is less than 2 g/dL; or, the process of purifying platelets comprises a procedure to remove plasma so that the concentration of fibrinogen in the ultra-pure platelet solution is less than 100 $\mu$g/mL.

5.  The method of claim 1, the platelet-containing solution is whole blood, and step (a) comprises:

    (a1) taking a unit of platelets-containing solution;
    (a2) performing a first sedimentation process, so that the platelet-containing solution is divided into a plasma layer, a buffy coat layer, and a red blood cell layer;
    (a3) removing the buffy coat layer and the red blood cells layer to obtain a plasma layer solution;
    (a4) performing a second sedimentation process to allow the platelets to settle and form a pellet;
    (a5) removing the supernatant and mix the pellet with a solvent to obtain the ultra-pure platelet solution.

6.  The method of claim 1, wherein, the activation procedure is a freezing and then thawing procedure (i.e., a freeze-thaw procedure), an addition of $Ca^{2+}$ procedure, an addition of thrombin procedure, an addition of collagen procedure, or an addition of ADP procedure, or any combination thereof.

7.  The method of claim 1, wherein, the activation procedure is a freezing and then thawing procedure (freeze-thaw procedure), and the freezing and then thawing procedure (freeze-thaw procedure) comprises:

    (b1) placing a container containing the ultra-pure platelet solution in liquid nitrogen to freeze the ultra-pure platelet solution; and
    (b2) raising the temperature of the container to thaw the frozen ultra-pure platelet solution.

8.  The method of claim 1, wherein

    (1) the content of PDGF-BB per gram (g) of growth factors-enriched dry powder is 10 pg ~ $3\times10^7$pg; or
    (2) the content of VEGF per gram (g) of growth factors-enriched dry powder is $2 \times 10^2$pg ~ $3\times10^6$pg; or
    (3) the content of FGF per gram (g) of growth factors-enriched dry powder is 10 pg ~ $1\times10^4$pg; or
    (4) the content of TGF-$\beta$1 per gram (g) of growth factors-enriched dry powder is $2\times10^5$pg ~ $1.2\times10^7$pg; or
    (5) the content of EGF per gram (g) of growth factors-enriched dry powder is $1\times10^2$pg~$6\times10^5$pg.

9.  The method of claim 2, wherein, the freeze-drying procedure comprises lowering the temperature to less than or equal to -35°C and lowering the pressure to less than or equal to 80 mTorr.

10. The method of claim 1, wherein, the platelet-containing solution is whole blood, apheresis platelet, leukocytes-reduced platelets apheresis, or Platelet-Rich Plasma (PRP), or any combination thereof; or,

the platelet-containing solution is derived from an autologous blood sample or an allogeneic blood sample, or a combination thereof.

11. The method of claim 5, the solvent in step (a5) is sterile saline, water for injection, 0.45% NaCl, 4.5% hypertonic saline, sterile warm spring water, or isotonic saline; or, the platelet concentration in the ultra-pure platelet solution in step (a5) is $1\times10^9$ cells/mL to $10\times10^9$ cells/mL.

12. The method of claim 1, wherein, step (c) comprises:

(c1) performing a sedimentation process to allow a platelet-associated structure to settle and form a platelet-associated structure pellet;
(c2) removing the platelet-associated structure pellet to obtain the growth factors-enriched solution.

13. A growth factors-enriched dry powder that is prepared by a preparation method, and the preparation method comprises:

(a) taking a unit of platelet-containing solution and perform a process of purifying platelets to obtain an ultra-pure platelet solution;
(b) performing an activation procedure to allow platelets activation in ultra-pure platelet solution and release growth factors to obtain a platelet growth factor extract solution;
(c) performing a platelet-associated structure removal procedure to obtain a growth factors-enriched solution; and
(d) performing the freeze-drying procedure on growth factors-enriched solution in order to obtain the growth factors-enriched dry powder, wherein the freeze-drying procedure comprises lowering the temperature to less than or equal to -30°C and lowering the pressure to less than or equal to 100 mTorr;

wherein, the content of PDGF-BB per gram (g) of growth factors-enriched dry powder is 10 pg ~$3\times10^7$ pg.

14. The growth factors-enriched dry powder of claim 13, wherein, the activation procedure is a procedure of freezing and then thawing (freeze-thaw procedure); and the procedure of freezing and then thawing (freeze-thaw procedure) comprises:

(b1) placing a container containing the ultra-pure platelet solution in liquid nitrogen to freeze the ultra-pure platelet solution; and
(b2) raising the temperature of the container to thaw the frozen ultra-pure platelet solution.

15. Use of the growth factors-enriched dry powder of claim 13 in the preparation of a pharmaceutical composition or health composition for administrating an effective dose to a specific site in an individual to relieve the degree of inflammation or injury of the site.

16. The use of claim 15, wherein the lysis procedure is a procedure of freezing and then thawing (freeze-thaw procedure); and the procedure of freezing and then thawing (freeze-thaw procedure) comprises:

(b1) placing a container containing the ultra-pure platelet solution in liquid nitrogen to freeze the ultra-pure platelet solution; and
(b2) raising the temperature of the container to thaw the frozen ultra-pure platelet solution.

17. The use of claim 15, the process of purifying platelets in step (a) further comprises a procedure for removing white blood cells so that the number of white blood cells per milliliter (mL) of the ultra-pure platelet solution is less than $10^6$ cells.

18. The use of claim 15, the process of purifying platelets in step (a) further comprises a procedure for removing plasma so that the concentration of albumin in the ultra-pure platelet solution is less than 3 g/dL; or, the process of purifying platelets further comprises a procedure for removing plasma so that the concentration of globulin in the ultra-pure platelet solution is less than 2 g/dL; or, the process of purifying platelets further comprises a procedure for removing plasma so that the concentration of fibrinogen in the ultra-pure platelet solution is less than 100 μg/mL.

19. The use of claim 15, wherein

(1) the content of VEGF per gram (g) of growth factors-enriched dry powder is $2\times10^2$ pg $\sim3\times10^6$ pg; or

(2) the content of FGF per gram (g) of growth factors-enriched dry powder is 10 pg - $1\times10^4$ pg; or

(3) the content of TGF-$\beta$1 per gram (g) of growth factors-enriched dry powder is $2\times10^5$pg $\sim 1.2\times10^7$pg; or

(4) the content of EGF per gram (g) of growth factors-enriched dry powder is $1\times10^2$pg$\sim6\times10^5$pg.

20. The use of claim 15, wherein the growth factors-enriched dry powder is used in the manufacture of a pharmaceutical compositions or health compositions to relieve the degree of inflammation or injury of a respiratory tract, skin, muscle, tendon, bone, joint or ligament of the individual.

S11

Taking a unit of platelet-containing solution and performing a process of purifying platelets to obtain an ultra-pure platelet solution

S12

Performing an activation procedure to allow platelets activation in ultra-pure platelet solution and release growth factors to obtain a platelet growth factor extract solution

S13

Performing a procedure to remove a platelet-associated structure to obtain a growth factor enriched solution

S14

Subjecting the growth factor enriched solution to a drying process to obtain the growth factor enriched dry powder

Figure 1

Figure 2A

Figure 2B

Figure 3A

Figure 3B

Figure 4

Figure 5

Figure 6

Europäisches
Patentamt

European
Patent Office

Office européen
des brevets

# EUROPEAN SEARCH REPORT

**Application Number**

EP 23 16 3851

## DOCUMENTS CONSIDERED TO BE RELEVANT

| Category | Citation of document with indication, where appropriate, of relevant passages | Relevant to claim |
|---|---|---|
| X | WO 2014/027362 A1 (KASIAK RES PVT LTD [IN]) 20 February 2014 (2014-02-20) * page 8, paragraph 5; claims 1-3, 8, 11 * * page 2, paragraph 2 * * page 18, paragraph 2 * * examples 1-6 * ----- | 1-20 |
| X | KIEB MATTHIAS ET AL: "Platelet-Rich Plasma Powder: A New Preparation Method for the Standardization of Growth Factor Concentrations", AMERICAN JOURNAL OF SPORTS MEDICINE., vol. 45, no. 4, 13 December 2016 (2016-12-13), pages 954-960, XP093093623, WALTHAM, MA. ISSN: 0363-5465, DOI: 10.1177/0363546516674475 Retrieved from the Internet: URL:http://journals.sagepub.com/doi/full-xml/10.1177/0363546516674475> * abstract * * page 954, paragraph 1 * * page 955, left-hand column, paragraph 3 * * page 955, right-hand column, paragraph 1 - paragraph 3 * * page 956, left-hand column, paragraph 1 * ----- | 1-4,6,8, 10,12-14 |
| X | WO 2007/098127 A2 (WOUND CARE PARTNERS LLC [US]; GANDY JAMES [US]) 30 August 2007 (2007-08-30) * claims 1-4 * * page 0018 * * examples 1-8 * ----- -/-- | 1-6,8, 12-20 |

**CLASSIFICATION OF THE APPLICATION (IPC)**

INV.
A61K35/19
A61K8/98
A61K9/19
A61K38/18
F26B5/06
A61P43/00

**TECHNICAL FIELDS SEARCHED (IPC)**

A61K
A61P
F26B

The present search report has been drawn up for all claims

| Place of search | Date of completion of the search | Examiner |
|---|---|---|
| Munich | 28 October 2023 | Böhmerova, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 23 16 3851

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/213238 A1 (GANDY JAMES BENNIE [US] ET AL) 4 September 2008 (2008-09-04) * claims 1-2, 6-7 * * left-hand column, paragraph 3 - page 3 * * example 1 * ----- | 1-4,6,8, 10,13-20 | |
| X | WENG HSIN-PEI ET AL: "Enhanced Platelet-Rich Plasma (ePRP) Stimulates Wound Healing through Effects on Metabolic Reprogramming in Fibroblasts", INTERNATIONAL JOURNAL OF MOLECULAR SCIENCES, vol. 22, no. 23, 23 November 2021 (2021-11-23), page 12623, XP093094270, DOI: 10.3390/ijms222312623 * abstract * * page 2, paragraph 2 - paragraph 3 * * page 3, paragraph 1 * * page 14, paragraph 1 * * figure 1 * ----- | 1-6,8, 10,12-20 | |
| A | BEITIA MAIDER ET AL: "Platelet Lysate Nebulization Protocol for the Treatment of COVID-19 and Its Sequels: Proof of Concept and Scientific Rationale", INTERNATIONAL JOURNAL OF MOLECULAR SCIENCES, vol. 22, no. 4, 12 February 2021 (2021-02-12), page 1856, XP055980820, Basel, CH ISSN: 1661-6596, DOI: 10.3390/ijms22041856 * page 3, paragraph 2; table 1 * * page 12, paragraph 3 - paragraph 4 * ----- | 1-20 | **TECHNICAL FIELDS SEARCHED (IPC)** |

The present search report has been drawn up for all claims

| Place of search | Date of completion of the search | Examiner |
|---|---|---|
| Munich | 28 October 2023 | Böhmerova, Eva |

EPO FORM 1503 03.82 (P04C01)

## ANNEX TO THE EUROPEAN SEARCH REPORT
## ON EUROPEAN PATENT APPLICATION NO.

EP 23 16 3851

This annex lists the patent family members relating to the patent documents cited in the above-mentioned European search report.
The members are as contained in the European Patent Office EDP file on
The European Patent Office is in no way liable for these particulars which are merely given for the purpose of information.

28-10-2023

| Patent document cited in search report | | Publication date | Patent family member(s) | | Publication date |
|---|---|---|---|---|---|
| WO 2014027362 | A1 | 20-02-2014 | EP | 2884992 A1 | 24-06-2015 |
| | | | ES | 2639561 T3 | 27-10-2017 |
| | | | US | 2015224173 A1 | 13-08-2015 |
| | | | WO | 2014027362 A1 | 20-02-2014 |
| WO 2007098127 | A2 | 30-08-2007 | US | 2006142198 A1 | 29-06-2006 |
| | | | US | 2012114760 A1 | 10-05-2012 |
| | | | WO | 2007098127 A2 | 30-08-2007 |
| US 2008213238 | A1 | 04-09-2008 | US | 2005191286 A1 | 01-09-2005 |
| | | | US | 2008213238 A1 | 04-09-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ISABLEANDIA, NICOLA MAFFULLI.** Platelet-rich plasma for managing pain and inflammation in osteoarthritis. *Nature Review Rheumatology,* 2013, vol. 15, 721-730
- **PENGCHENG XU et al.** Platelet-rich plasma accelerates skin wound healing by promoting re-epithelialization. *Burns & Trauma,* 2020
- **LINFENG PIAO ; HYUNGMIN PAR ; CHRIS HYUNCHUL JO.** Theoretical prediction and validation of cell recovery rates in preparing platelet-rich plasma through a centrifugation. *PLOS ONE,* 2017, 12
- **SEAN R DOWNING ; GIANNOULA L KLEMENT.** Isolation and proteomic analysis of platelets by SELDI-TOF MS. *Methods in Molecular Biology,* 2012, vol. 818, 153-170
- **ZU-YAO CHANG ; GHEORGHE A.M. POP ; GERARD C.M. MEIJER.** *A NOVEL MODEL OF BLOOD IMPEDANCE FOR INDIRECT VISCOSITY MEASUREMENT,* 2004